# EUROPEAN PATENT APPLICATION

(11) **EP 0 834 556 A1**
(43) Date of publication of application: **08.04.1998**
(21) Application number: 96202686.0
(22) Date of filing: 25.09.1996
(51) Int. Cl.: C12N 5/00, A61K 38/57, C07K 14/81

(54) **Methods for culturing cells**

(71) Applicant: Academisch Ziekenhuis Leiden, 2300 RC Leiden (NL)
(72) Inventor: The designation of the inventor has not yet been filed
(74) Representative: Smulders, Theodorus A.H.J., Ir.

(57) **Abstract**

The invention relates to methods for culturing cells, in particular to the expansion of hematopoietic cells, specifically stem cells and/or progenitor cells.

These cells can be inhibited in their outgrowth by the presence in the medium of proteases.

Methods to avoid this inhibition and related uses of this finding are disclosed.

## Description

The present invention relates to the field of biotechnology, in particular cell biology and specifically to cell cultures.

Cells taken from an organism can, under certain conditions, be kept alive and multiplied by culturing. Culturing means that the cells are brought into a container, usually a flask, a bottle, a tank or the like, together with a fluid which is physiologically acceptable for said cells and which contains nutrients and factors allowing the cells to grow (expand).

This so called medium is usually synthetic and therefore well defined except for the growth factors which are often at least partially supplied in the form of mammalian serum (be it of bovine, horse or human origin). Many different kinds of cells can and have been cultured, ranging from plant cells to insect cells and from amphibian to mammalian cells. Of course many micro-organisms such as bacteria, bacilli and other prokaryotes as well as yeast and filamentous fungi can and have also been cultured. Cells can be divided into two groups for culturing; those that need to adhere in order to be able to be cultured and those that grow suspended in the medium.

Cells are cultured for many different reasons. Among these many reasons are the production of (clinically) useful proteins which are made and preferably excreted by the cells in question, obtaining a larger number of cells which themselves can be used (therapeutically), etc.

If substances derived from cell culture or cells produced in cell culture have to be used therapeutically, this calls for a thorough purification of said substances or cells. Removing a well defined synthetic medium is not so much of a problem, but removing undefined mixtures of many different molecules such as present in serum does present a problem. The therapeutic use of the products requires maximally defined culture media.

Serum contains many undefined proteins and other substances that may be harmful for the recipient of the therapy. However, many cells need this undefined mixture containing many factors and nutrients in order to survive and expand.

It would be a very significant improvement if the factors from serum which are essential for supporting growth of cells in culture could be identified or if ways could be developed for culturing cells without serum. The present invention now provides a factor which obviates the use of serum in certain cell cultures. The present invention thus provides a method for culturing cells which can be inhibited by the presence of proteases comprising bringing the cells into a container suitable for culturing together with a culture medium and growth factors, whereby a protease inhibitor is added to said culture medium. We have found that many cells, especially hematopoietic cells, in particular stem cells and/or progenitor cells, are sensitive to the presence of proteases in the culture medium. Purified hematopoietic progenitor cells c.q. stem cells can be cultured in the presence of multiple hematopoietic growth factors in the presence as well as in the absence of serum. In the presence of bone marrow accessory cells (in particular mononuclear cells) growth of hematopoietic progenitor cells is suppressed in the absence of serum. This suppression can be neutralized by the presence of serum, or conditioned medium from 5637 bladder carcinoma cells. Purification from this conditioned medium showed that the neutralizing capacity was due to the presence of a serine protease inhibitor antileukoprotease (ALP). Purified as well as recombinant ALP were capable of replacing both serum and 5637 illustrating that the suppressive effect of bone marrow mononuclear cells can be neutralized by serum or 5637, probably due to the presence of a serine protease inhibitor in these fluids. The main serine protease inhibitor in serum is α1-PI. It was demonstrated that α1-PI was similar to ALP capable of neutralizing the suppressive effect of bone marrow mononuclear cells. In the experimental part the invention is explained in more detail relating to culturing hematopoietic cells, together with a serine protease inhibitor (antileukoproteinase). It is clear that other serine protease inhibitors (serpins are preferred) will work as well and also that the invention can be applied to other types of cells which are protease sensitive in the sense that small amounts inhibit cell growth. It is also clear that only a functional part of the protease inhibitor needs to be present, meaning that only the proteinase inhibiting activity is necessary.

This may be achieved by a derivative or a fragment of the original serpin or protease inhibitor. It may even be achieved by anti-idiotypic antibodies or peptidomimetics.

The method is of course preferred for processes leading to clinical applications or products and is in the case of cells thus preferred to be used with human cells and in the case of therapeutic substances to be derived from the cells to be used for human substances or substances compatible with the human system.

A particular application of interest is in bone marrow transplantation wherein stem cells and/or progenitor cells are expanded *in vitro* before they are introduced to repopulate a human hematopoietic and immune system.

Stem cells and progenitor cells are often cultured in the presence of bone marrow mononuclear cells. These mononuclear cells are a source of proteases, in particular elastase (human neutrophil elastase (HNE)) and cathepsin-G. We demonstrated that on purified hematopoietic progenitor cells (HPC) elastase and cathepsin-G were capable of suppression of the outgrowth of HPC in the presence of multiple hematopoietic growth factors in the absence of serum. This suppressive effect could also be neutralized by α1-PI or ALP. These experiments illustrate that the likely mechanism of neutralization of inhibition by serum or 5637 conditioned medium is due to the neutralization by serine protease inhibitors of proteases like elastase and cathepsin-G.

In addition to the hematopoietic progenitor cells, the T cell line CTLL-2 either in wild type configuration, or transfected with a G-CSF receptor, could be inhibited in the presence of the relevant growth factors by elastase or cathepsin-G.

Similar to bone marrow hematopoietic progenitor cells, this suppression could be neutralized by α1-PI.

There is a further important consequence of the finding which is an important part of this invention, i.e. the relevance of serineprotease inhibitors in the culturing of cells, in particular hematopoietic (stem and/or progenitor) cells. This consequence leads to a clinical application of in particular elastase and cathepsin-G antagonists, which may be serpins or any other molecule inhibiting the action of these proteinases.

It has been disclosed that myelocytic leukaemia cells produce signifantly increased amounts of elastase (found complexed with α₁-PI (26). It is also known that in patients suffering from such leukemias the normal production of healthy bone marrow cells is suppressed. Knowing now, because of the present invention that elastases inhibit the outgrowth of stem cells and/or progenitor cells, it is clear that a clinical application of serpins or other antagonists of these elastases is within reach. The normal production of healthy bone marrow cells may be restored, or at least be less inhibited by administration of elastase inhibitors to patients suffering from myeloid leukemias.

### Detailed description.

Serum contains many growth factors and nutrients that stimulate colony formation of hematopoietic progenitor cells (HPC) in semisolid cultures. In the absence of serum no proliferation of HPC could be obtained in semi-solid medium cultures of partially purified bone marrow cells in the presence of multiple hematopoietic growth factors, insulin, cholesterol and purified clinical grade human albumin. This appeared to be due to a suppressive activity induced by monocyte- and T lymphocyte-depleted accessory cells on CD34 positive HPC. Serum-free conditioned medium from the bladder carcinoma cell-line 5637 could replace serum to support the growth of HPC in these cultures. After gel filtration and Reverse-Phase High Performance Liquid Chromatography (RP-HPLC) of 5637 supernatants, this activity could be attributed to a 15kD protein that was further identified by NH₂-terminal sequence analysis as the serine proteinase inhibitor Antileukoproteinase (ALP). The growth-supportive activity from the 5637 conditioned medium, and the (partially) purified fractions could be completely neutralized by a polyclonal rabbit IgG antibody against human ALP (huALP). Similar supportive effects on the growth of HPC could be obtained in the presence of recombinant huALP (rALP). We demonstrated that the COOH-terminal domain of ALP containing the proteinase inhibitory activity was responsible for this effect. α-I proteinase inhibitor was capable of similar support of *in vitro* HPC growth. These results illustrate that proteinase inhibitors play an important role in cell culture and in particular in the *in vitro* growth of hematopoietic cells by the neutralization of proteinases produced by bone marrow accessory cells. This may be of particular relevance for *in vitro* expansion of human hematopoietic stem cells in serum-free media.

Characterization of hematopoiesis in man is dependent on the *in vitro* analysis of hematopoietic progenitor cells (HPC).

HPC can be studied using semi-solid medium cultures, and their potential for proliferation and differentiation can be characterized by their ability to form colonies of hematopoietic cells in these cultures. The growth and differentiation of HPC in colony assays depends on the presence of hematopoietic growth factors (HGF) in the cultures (1). These HGF may be added to the culture medium or produced by accessory cells or HPC as paracrine or autocrine growth factors, respectively. In addition, factors that are present in serum including insulin, cholesterol or albumin are critical for the *in vitro* growth of HPC (2-7). Bovine serum albumin (BSA), frequently used as a source of albumin, is usually not more than 95-99 % pure because of its strong protein binding properties (8,9). Since in serum-free colony assays BSA is often used at relatively high concentrations, residual undefined proteins may significantly contribute to the growth supportive potential of BSA. Pilot studies in our laboratory indicated that in contrast to these BSA preparations highly purified concentrates of clinical grade human albumin did not support the proliferation of HPC from monocyte- and T lymphocyte-depleted mononuclear bone marrow cells in semi solid medium cultures in the presence of HGF, cholesterol and insulin. In this study, we analyzed the mechanisms of this finding and showed that this was due to a suppressive effect of monocyte- and lymphocyte-depleted bone marrow accessory cells on CD34 purified HPC. We investigated which serum factor in addition to human albumin, insulin and cholesterol was responsible for supporting the *in vitro* proliferation of bone marrow derived HPC. We found that a 15 kD protein, present in conditioned medium from the bladder carcinoma cell line 5637 could replace serum. This protein was identified as the serine proteinase inhibitor antileukoproteinase (ALP).

### MATERIALS AND METHODS

### Cell preparations

After informed consent, bone marrow samples were obtained from healthy donors. The cells were centrifuged on Ficoll Isopaque (density 1.077 g/cm³, 20 min., 1,000 g) and mononuclear cells were depleted of monocytes and T lymphocytes as described previously (10). These enriched bone marrow samples were cryopreserved and stored in liquid nitrogen. Immediately before use, the cells were thawed rapidly in a waterbath of 37°C, washed once in cold (O°C) RPMI-medium (Hyclone, Logan, UT) supplemented with 20 % (v/v) Fetal Bovine Serum (FBS, Boehringer, Mannheim, Germany), and twice with cold RPMI-medium, supplemented with 0.1 % (w/v) clinical grade Human Serum Albumin (HSA, Central Laboratory of the Bloodtransfusion Services, Amsterdam, The Netherlands). The cells were resuspended in Iscove's Modified Dulbecco's Medium (IMDM, Hyclone), supplemented with 0.1 % (w/v) HSA. In some experiments highly purified CD34 positive cells were isolated from the monocyte- and T lymphocyte-depleted bone marrow cells. The CD34 positive cells were either selected after staining with FITC conjugated CD34 specific monoclonal antibodies (HPCA-2, Becton Dickinson, Mountain View, CA), and sorting with a FACStar PLUS (Becton Dickinson) or isolated after incubation with magnetic Dynabeads M-450 (Dynal Inc., Lake Success, NY) coated with a primary antibody specific for the CD34 Ag. After magnetizing the CD34 positive cells the non-binding population was harvested as the CD34 negative. accessory cells. The CD34 positive cells were recovered after incubation with a sheep polyclonal antibody anti CD34 Fab IgG (Detachabead CD34, Dynal Inc) resulting in > 80 % pure CD34 positive cells.

### Cytokines and antibodies

All cytokines used in this study were recombinant human proteins and used at plateau concentration to induce optimal colony growth. The cytokines and final concentrations used were: Stem Cell Factor (SCF) (50 ng/ml), granulocyte-colony stimulating factor (GCSF) (10 ng/ml), basic fibroblast growth factor (bFGF) (10 ng/ml), and epidermal growth factor (EGF) (10 ng/ml) provided by Amgen (Thousand Oaks, CA); IL-3 (50 ng/ml), IL-11 (10 ng/ml), Monocyte-CSF (M-CSF) (1,000 U/ml) and granulocyte/monocyte-CSF (GM-CSF) (10 ng/ml) were provided by Genetics Institute (Cambridge, MA); IL-lα (10 ng/ml), IL-5 (10 ng/ml), IL-12 (10 ng/ml), were gifts from Hoffman la Roche (Nutley, NJ); IL-4 (500 U/ml) and IL-7 (10 ng/ml) were gifts from Immunex Corporation (Seattle, WA), IL-6 (1 ng/ml) and IL-8 (10 ng/ml) were gifts from Sandoz (Basel, Switzerland); platelet derived growth factor (PDGF, 30 ng/ml) was obtained from Life Technology BRL (Grand Island, NY); insulinlike growth factors (IGF-I, IGF-II, 250 ng/ml) were obtained from Genzyme Corporation (Boston, MA), and EPO (1 U/ml) was a gift from Cilag (Herentals, Belgium). IL-9 (1,000 U/ml) was provided by Ludwig Institute for Cancer (Brussel, Belgium), IL-10 (10 ng/ml) was obtained from Schering-Plough Research Institute (Kenilworth, NJ) and IL-2 (300 U/ml) was a gift from Roussel Uclaf (Paris, France). Recombinant human antileukoproteinase (rALP) was a generous gift from Dr. R.C. Thompson (Synergen Inc., Boulder, CO). The separated NH₂-terminal and the COOH-terminal domains of ALP, designated ALP-D-1 and ALP-D-2 respectively, were kindly donated by Dr. G. Steffens (Grünentahl, Aachen, BRD), and were used at equivalent molar concentrations of rALP. α-I proteinase inhibitor (α-I PI), used at a final concentration of 0.2 to 20 µg/ml, was obtained from Cutter Biologic (Hartford, Connecticut).

A neutralizing anti-huALP antibody was prepared in rabbits.

The antibody has been found to have a high titer and specificity for human antileukoproteinase (11).

### Clonogenic assay

Semi solid medium cultures were performed in sixfold in flatbottomed 96 well microtiterplates (Greiner, Alphen a/d Rijn, the Netherlands) in aliquots of 0.1 ml per well, containing 10³ monocyte- and T lymphocyte-depleted mononuclear bone marrow cells or 150 purified CD34 positive cells per well. The culture medium consisted of IMDM, supplemented with 0.6 % (w/v) HSA, 20 µg/ml Cholesterol (Sigma C-7402), 10 µg/ml Insulin (Sigma I-4011), 5xlO⁻⁵ M β-Mercaptoethanol, 0.47 mg/ml Human Transferrin (Behringwerke AG, Marburg, Germany) saturated with FeCl₃.6H₂0, Methylcellulose (Methocel 4000 cps, Fluka, Freiburg, Germany) at a final concentration of 1.1 % (w/v) and a mixture of cytokines: GM-CSF (10 ng/ml), G-CSF (10 ng/ml), IL-3 (50 ng/ml), SCF (50 ng/ml) and EPO (1 IU/ml).

All cytokines were present at saturating concentrations. This culture medium was supplemented either with control medium, human AB serum from healthy blooddonors (10 % (v/v)), prescreened for alloantibodies and inactivated for 30 min. at 56°C, or a conditioned medium (CM), produced by the human bladder carcinoma cell-line 5637 in the absence of serum, or (partially) purified fractions from this CM or proteinase inhibitors. Purified CD34 positive cells were cultured in the absence or presence of increasing numbers of monocyte- and T lymphocyte-depleted bone marrow accessory cells. In some experiments, to neutralize the biologic activity of these supplements a 2 h incubation at 37°C and 5 % C0₂ in the presence of neutralizing concentrations of anti-huALP antibodies was performed before initiation of the BM culture.

After 12 days of incubation in a fully humified atmosphere of 5% C0₂ at 37°C, colonies were counted. Granulocyte-macrophage colonies (CFU-GW were defined as aggregates of at least 50 cells and erythroid colonies (BFU-E) as hemoglobinized. bursts. To calculate the HPC growth in the partially purified bone marrow fractions the colony growth induced by the culture medium alone was considered background and therefore in these experiments subtracted from the colony growth induced by AB serum, CM or purified CM fraction. BFU-E and CFU-GM growth in the presence of 10% (v/v) AB serum was considered 100% growth (control culture). Colony growth was expressed as percentage growth of the control culture.

### Production of conditioned medium

The human bladder carcinoma cell-line 5637 (12,13) was used to produce a serum-free conditioned medium (CM). Confluent adherent cells were washed three times with RPMI-1640 and incubated for 24 h in RPMI-1640 serum-free medium, supplemented with 0.6% HSA. Then, the cells were washed three times with RPMI-1640 and cultured in IMDM, supplemented with 5xlO⁻⁵ M β-Mercaptoethanol and 0.47 mg/ml human Transferrin, saturated with FeClO₃.6H₂0. After 48 h. the culture supernatant was harvested. The crude supernatant was centrifuged (15 min., 500 g, 20^{°}C), irradiated (50 Gy) and frozen at -20^{°}C after addition of 0.1 % (w/v) HSA for stabilisation.

### Purification of conditioned medium

Crude supernatant from 5637 cells was incubated for 2 h at 4^{°}C with Controlled Pore Glass (CPG) beads (Sigma PG-350-200), for adsorption at pH 7.0 (14-16). The CPG beads were washed with Dulbecco's modified phosphate buffered saline (PBS) and 0.01 M glycine/HCl pH 3.5. The protein fraction was eluted by carefully stirring with 0.3 M glycine/HCl pH 2.0 (1/10 of original crude volume), concentrated and neutralized by dialysis against PBS containing 10 % (w/v) polyethylene glycol (PEG 20.000) (Serva, Heidelberg, FRG). For further fractionation of proteins a gel filtration was performed on Ultrogel ACA 54 (LKB, Bromna, Sweden) using a 2.6-cm x 100-cm column and a flow rate of 22 ml/h. Bovine serum albumin (Mᵣ 67,000), ovalbumin (Mᵣ 45,000), chymotrypsinogen (Mᵣ 25,000) and lysozyme (Mᵣ 14,300) were used as molecular mass markers.

Fractions of 5.5 ml were collected by elution with 18% ethylene glycol buffer in 1.55 M NaCl, 8 mM phosphate, pH 7.2.

The protein concentration was analyzed by a Coomassie blue/G-250-binding assay, using the Bio-Rad (Richmond, CA) commercial kit and bovine serum albumin as a standard. Fractions from the Ultrogel column, that could support colony growth of HPC, were supplemented with Tween 20 (0.01% v/v), concentrated on a centricon-3 filter (Amicon Inc., Beverly, MA) and further fractionated by Reverse Phase HPLC (RP-HPLC). The concentrate was injected on a C8-Aquapore RP-300 (2.1 x 220 mm) column (Applied Biosystems Inc. Foster City, CA), equilibrated with 0.1 % trifluoroacetic acid (TFA). Proteins were eluted at a flow rate of 0.4 ml/min. with a linear gradient of, acetonitril (0-80% in 80 min.) in 0.1% TFA. Fractions of 400 µl were collected in tubes, containing 0.01 % (final concentration) Tween 20 and the absorption at 220 nm was measured.

Active RP-HPLC fractions, containing low protein concentrations were analyzed for purity by SDS/PAGE. Samples (20 µl) were loaded on to a polyacrylamide gel (reducing conditions), run simultaneously with molecular mass markers and proteins were silver stained. The electrophoretically pure fractions were finally analyzed on a 477A protein sequencer with on-line detection of the phenylthiohydantoin AA in a 120A analyzer (Applied Biosystems) (17). Chemical digestion of protein was obtained with 75% formic acid at 37^{°}C for 50 h (17).

### RESULTS

### Influence of serum and accessory cells on HPC growth

In cultures containing 10 % AB serum and HGF (IL-3, GM-CSF, G-CSF, SCF, EPO) the absolute numbers of HPC per 10³ monocyte- and T lymphocyte-depleted bone marrow cells were 21±2 CFU-GM and 20±2 BFU-E (n=8).

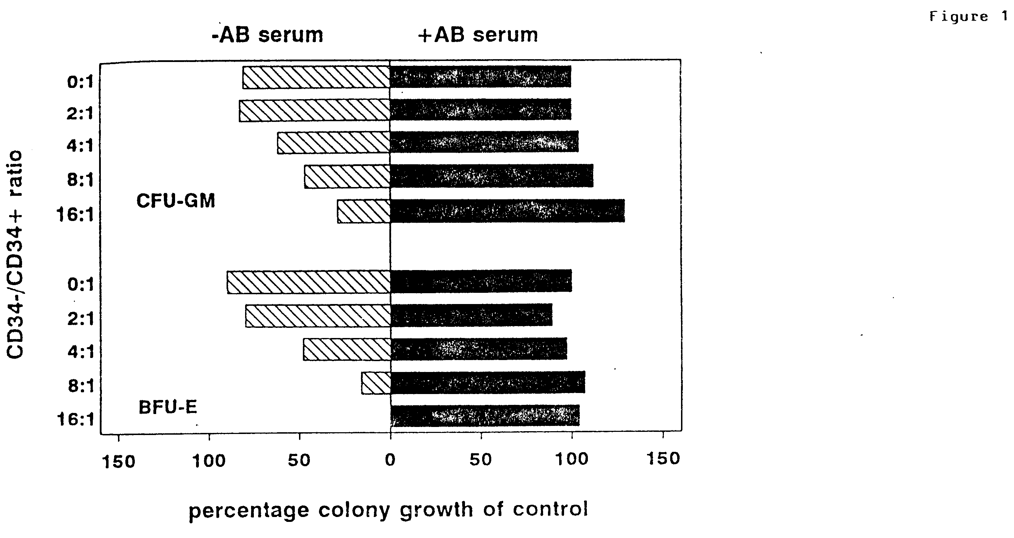

Replacement of AB serum by HSA (0.6% w/v), cholesterol (20 µg/mi) and insulin (10 µg/ml) resulted in only 4±1 GM-colonies and no BFU-E growth per 10³ monocyte- and T lymphocyte-depleted mononuclear bone marrow cells plated (background colony growth). In contrast, in cultures of purified CD34 positive cells HPC growth in the presence of 10% AB serum yielded 16±1 CFU-GM and 10±1 BFU-E per 150 cells plated, while in the absence of AB serum 9±2 CFU-GM and 6±1 BFU-E were cultured (n=10). Figure 1 shows that addition of monocyte- and T lymphocyte-depleted CD34 negative bone marrow cells to purified CD34 positive cells resulted in a dose-dependent inhibition of HPC growth in the absence, but not in the presence of AB serum, illustrating that serum contains factors capable of supporting HPC growth in the presence of bone marrow accessory cells.

### Influence of 5637 conditioned medium on HPC growth in the absence of serum

Replacement of AB serum by Interleukins (IL-1 through IL-12) separately or in various combinations, or growth factors (platelet derived growth factor (PDGF), basic fibroblast growth factor (FGF), epidermal growth factor (EGF), insulin like growth factors (IGF-I, IGF-II) or macrophage-colony stimulating factor (M-CSF)) in addition to the cytokines present in the culture medium, did not result in additive growth of colonies from monocyte- and T lymphocyte-depleted bone marrow cells (data not shown). However, addition of 3-10% (v/v) conditioned medium (CM), produced by 5637 cells in the absence of serum could support the colony growth up to levels similar to the growth observed in the presence of 10% AB serum. Replacement of AB serum by 10% (v/v) 5637 CM to the culture medium supported 136±12% of CFU-GM growth and 85±4% of BFU-E growth as compared to the control culture. These results show that in the presence of purified human albumin, 5637 cells produce factors that can support the growth of CFU-GM and BFU-E comparable to the effect of AB serum (table 1).

### Characterizaizon of the supportive factor from 5637 conditioned medium

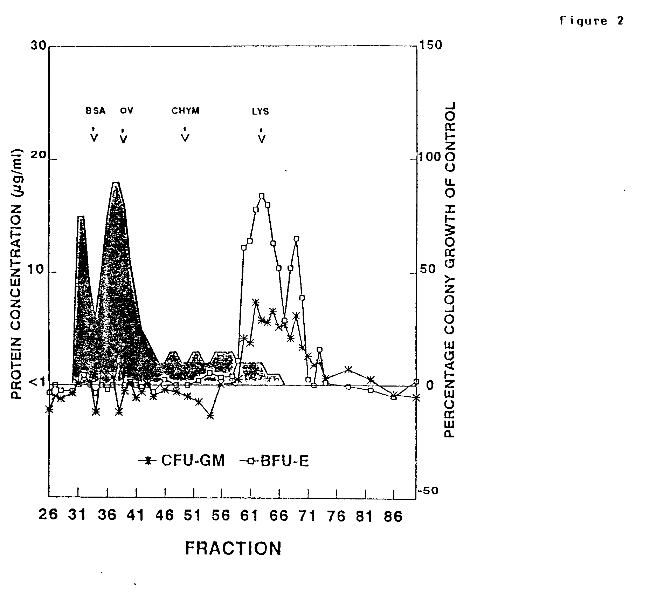

The protein fraction from the 5637 CM, concentrated by adsorption to and elution from the CPG beads, contained the colony growth supportive factor with a 5-fold higher concentration of activity compared to the crude 5637 supernatant (data not shown). After gel filtration of the CPG-eluate into 90 fractions the major protein peaks were recovered from fractions 30 to 40 (> 45 kD). As illustrated in figure 2, addition of 0.3% (v/v) of these high Mᵣ protein containing fractions to the culture medium did not result in an enhancement of colony growth. However, addition of 0.3% (V/V) of low Mᵣ Protein fractions 59 through 72, containing very low protein concentration (<1-2 µg/ml) supported the growth of both CFU-GM and BFU-E, with a maximal level of growth in the fractions 62 and 69 containing 10-15 kD protein.

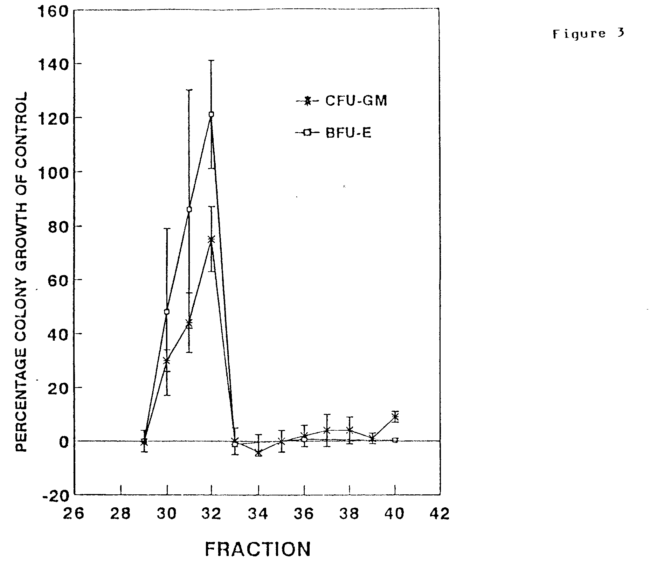

These fractions were chosen for further purification by RP-HPLC technique. After HPLC separation of fraction 62 the enhancing activity for both GM-colony growth and BFU-E growth was found in fractions 30, 31 and 32 (figure 3). Fraction 32 supported CFU-GM and BFU-E growth upto 75±12% and 125±20% of control cultures, respectively.

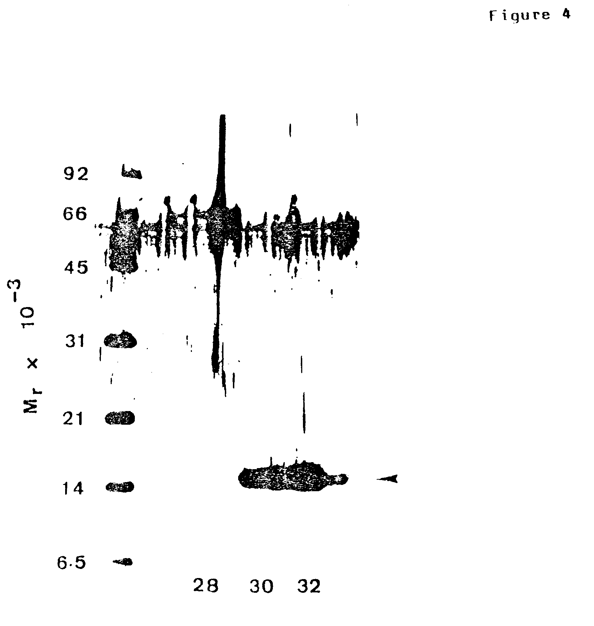

The HPLC fractions, which contained the highest growth supportive activity, were electrophoretically analyzed for purity by means of SDS/PAGE and the molecular mass was defined as shown in figure 4. The HPLC fractions 30, 31 and 32 from gel filtration fraction 62 showed a distinct protein band of 15 kD with the highest concentration in fraction 32(f-32).

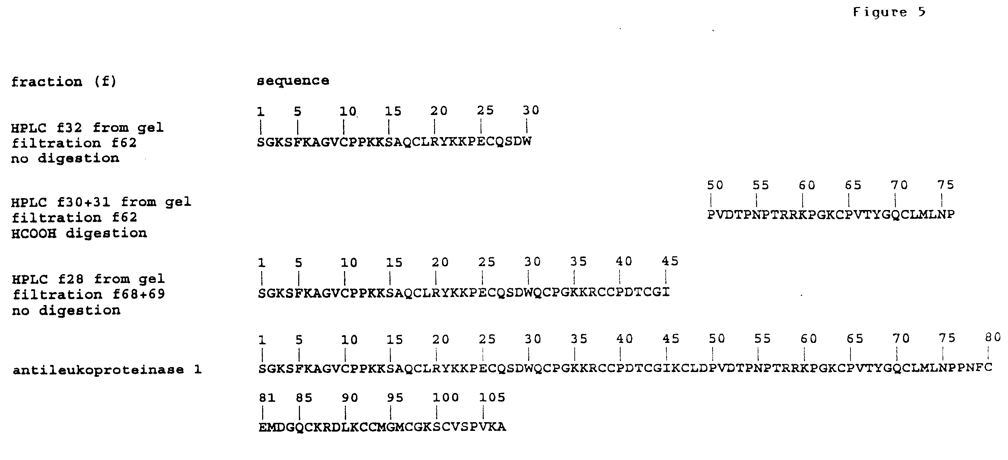

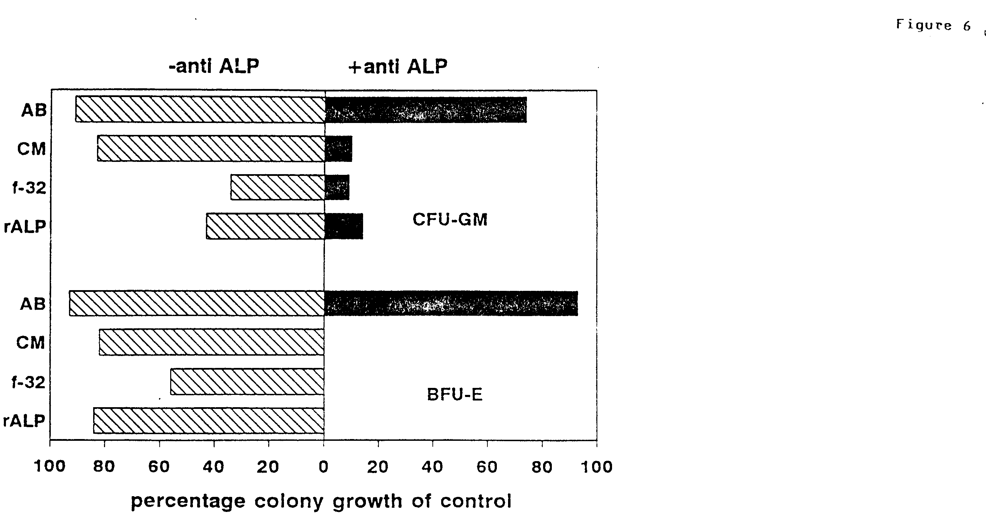

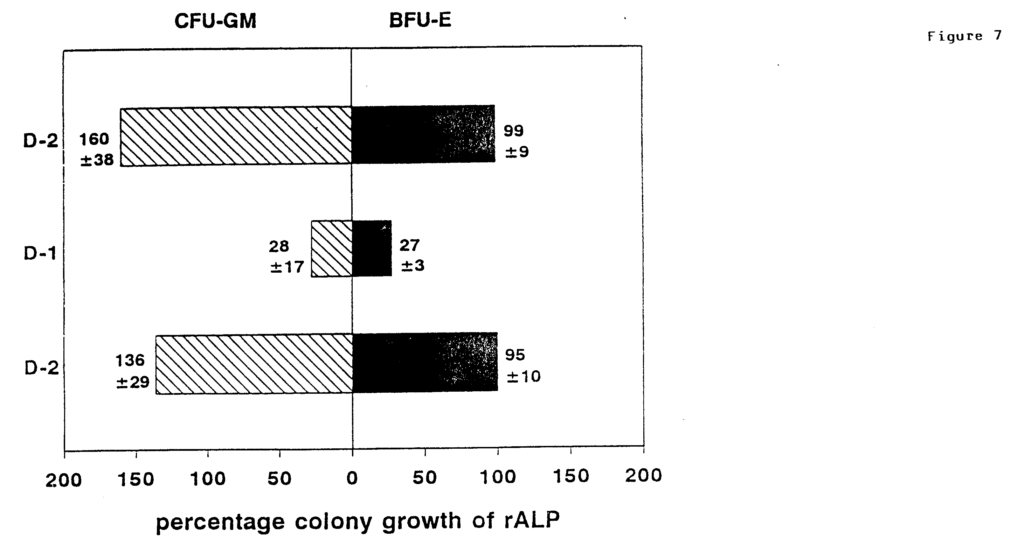

Using an automated protein sequencer, the first 30 NH₂-terminal amino acid residues of the protein were sequenced, and revealed complete structural identity with homology to the NH₂-terminal sequence of the serine proteinase inhibitor Antileukoproteinase (ALP). ALP consists of a total number of 107 residues. When purified natural protein was digested with formic acid, sequence analysis of the internal amino acids 50 to 76 showed again identity to ALP, confirming its complete homology to Antileukoproteinase (figure 5). Similarly, the second peak of activity from gel filtration (fractions 68 and 69) was purified by BPLC, yielding again a predominant 15 kD protein (fraction 28), which was also identified as ALP by NH₂-terminal sequence analysis (45 residues) (figure 5). As shown in figure 6, purified recombinant human ALP (rALP) could also support the growth of CFU-GM and BFU-E similar to 5637 CM or the purified 15 kD protein. Polyclonal anti-huALP antibodies could completely neutralize the biologic activity of 5637 conditioned medium (10% v/v), HPLC fraction-32 (0.1% v/v), and rALP (10 ng/ml). However, the support of AB serum (I % v/v) on HPC growth could not be neutralized by polyclonal anti-huALP antibodies (figure 6). Replacement of serum by the serine proteinase inhibitor α-1 PI (2 µg/ml), present in serum at a concentration as high as 25 µM, supported the growth of both CFU-GM and BFU-E progenitors similar to rALP. α-1 PI supported 69±20% of CFU-GM and 137±38% of BFU-E growth as compared to AB serum (n=3). Replacement of rALP by its domains ALP-D-1 or ALP-D-2 separately or in combination at molar concentrations equivalent to RALP, showed that the COOH-terminal domain ALP-D-2 contained the active site (figure 7).

### Influence of Antileukoproteinase on the suppressive effect of accessory cells on HPC growth in the absence of serum

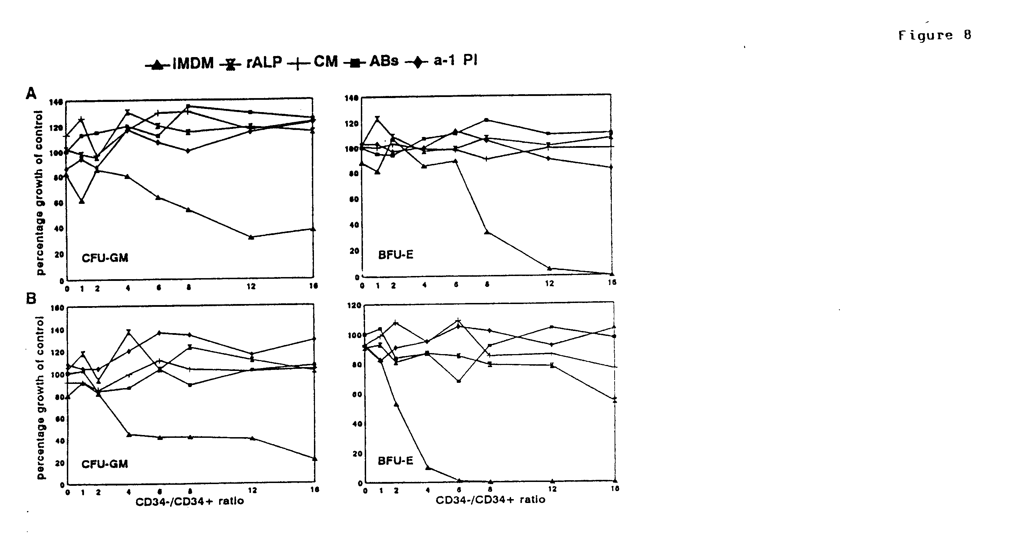

Monocyte- and T lymphocyte-depleted bone marrow cells were separated with Dynabeads into CD34 negative cells and a population, containing 80% CD34 positive cells. Figure 8 shows the colony growth of these purified CD34 positive cells in the absence or presence of increasing numbers of CD34 negative cells. In two separate experiments, it was shown that the inhibited growth of CFU-GM and BFU-E colonies, induced by the CD34 negative accessory cells in the absence of serum, was completely reconstituted by the addition of rALP (100 ng/ml) or CM (10% v/v). The proteinase inhibitor α-1 PI (2 µg/ml), present in serum at concentrations of 1-2 mg/ml, could also neutralize the suppression of both CFU-GM and BFU-E growth by CD34 negative cells in the absence of serum.

### DISCUSSION

in the presence of multiple hematopoietic growth factors, insulin, cholesterol and a highly purified clinical grade source of human albumin, we observed that *in vitro* HPC present in monocyte- or T lymphocyte-depleted bone marrow failed to proliferate and differentiate into mature colonies of myeloid and erythroid cells. In contrast, purified CD34 positive. cells proliferated in the absence of human AB serum.

Reconstitution experiments showed that accessory cells were responsible for suppression of growth of HPC in the absence of serum and that the addition of serum could restore HPC growth.

Similar to studies presented by several other investigators (1-7), in the presence of high concentrations of bovine serum albumin, growth of HPC was observed almost similar to the numbers of colonies in the presence of serum (data not shown).

Since BSA is usually less than 95-99% pure (8,9), unidentified serum factors bound to albumin may have influenced the results. Serum-free conditioned medium from the bladder carcinoma cell line 5637 could replace the need for serum or BSA in the semi-solid medium cultures to support the growth of HPC from normal bone marrow. Activity-based purification of this conditioned medium led to the isolation of a protein with a molecular mass of 15 kD (SDS/PAGE) which was identified by NH₂-terminal sequence analysis as Antileukoproteinase, a potent inhibitor of leukocyte elastase. The effect of this purified ALP on HPC growth could also be confirmed by recombinant human ALP. Both purified natural and recombinant ALP supported 100% of erythroid colony growth and more than 60% of the myeloid colony growth as compared to the control culture in the presence of 10% AB serum. In addition, we could completely neutralize the biologic activity from 5637-CM and purified natural ALP with a polyclonal IgG anti-huALP, illustrating that ALP was the factor essential for the *in vitro* HPC growth from monocyte- and T lymphocyte-depleted bone marrow cells in the presence of highly purified human albumin.

ALP is a 107 amino acid cationic protein consisting of two domains of similar architecture but with different inhibitory activities (18,19). The NH₂-terminal domain ALP-D-1 that is assumed to bind trypsin, could not support growth of HPC in the absence of serum. The COOH-terminal domain ALP-D-2, containing a strong inhibitory activity against chymotrypsin, neutrophil elastase and trypsin restored the colony growth indicating that the proteinase inhibitory activity of ALP was indeed responsible for the support of the HPC growth *in vitro*.

ALP, also known as secretary leukocyte proteinase inhibitor (SLPI), is present in a wide variety of mucous secretions and produced by epithelial-like cells (20). This local production of ALP with very high affinity for leukocyte elastase serves as a potential regulatory feedback mechanism to prevent epithelial damage caused by proteolytic proteinases (21).

Regulatory effects of ALP in hematopoiesis have not been described previously. Serum contains various serine proteinase inhibitors, of which α-1 proteinase inhibitor, and α2-macroglobulin are present at relatively high concentrations of 1.3 mg/ml and 1.7 mg/ml respectively (22); ALP has been found to circulate at a concentration of 50 ng/ml (21). When serum was replaced by α-1 PI, the main plasma proteinase inhibitor of neutrophil elastase (22), the growth support was comparable to rALP. These results support the evidence that the proteinase inhibitory activity is responsible for the observed effects. The high concentrations of other serine proteinase inhibitors may explain why anti-huALP antibodies did not abolish the stimulatory effect of serum.

Since the bone marrow accessory cells responsible for the suppression of growth of CD34 purified HPC were depleted of monocytes and T lymphocytes, the myeloid cells appeared to be the mediator cells for this effect. Probably, these myeloid cells secrete proteolytic proteinases like neutrophil elastase, that are capable of degrading cytokines, growth factor receptors or other proteins essential for the proliferation of HPC (23-25). ALP or other proteinase inhibitors present in serum apparently neutralized these proteolytic enzymes. The mechanisms by which ALP supported the proliferation of HPC *in vitro* may be at the cell membrane level by protecting cellular receptors from degradation, or by protection from degradation of proteins in the culture medium that are essential for HPC growth. In conclusion, we demonstrated that monocyte- and T lymphocyte depleted accessory bone marrow cells suppressed the *in vitro* growth of CD34 positive HPC in the absence of serum. The serine proteinase inhibitor ALP was capable of restoring the proliferation of HPC *in vitro* in the presence of multiple HGF, highly purified albumin, insulin and cholesterol. These results show that proteinase inhibitors are important for optimal *in vitro* growth of HPC and may be essential for *in vitro* expansion of human hematopoietic stem cells in serum-free medium.

### LEGENDS

### Figure 1

Influence of CD34 negative accessory cells on CFU-GM and BFU-E growth of purified CD34 positive cells in the presence of multiple HGF (G-CSF 10 ng/ml, GM-CSF 10 ng/ml, IL-3 50 ng/ml, SCF 50 ng/ml, EPO I U/ml) in the absence and presence of AB serum (10% v/v) (n=2). Colony growth of 150 CD34 positive cells plated per well, supplemented with increasing numbers of CD34 negative cells at ratio's up to 16:1, is expressed as percentage growth of control culture, obtained in the presence of 10% (v/v) AB serum in the absence of CD34 negative accessory cells. Absolute numbers of colonies in the control culture were 8±l CFU-GM and 11±1 BFU-E (mean±SE per 150 cells plated).

### Figure 2

Fractionation of HPC growth promoting activity from CPG-purified 5637 CM by gel filtration (Ultrogel ACA 54). The dark area represents the protein concentration per fraction.

The growth supportive activity for CFU-GM (x-x) and BFU-E (□-□) colonies from the monocyte- and T-lymphocyte depleted bone marrow MNC was analyzed in the presence of multiple HGF in the absence of serum (n=4). Colony growth supported by each column fraction at a final concentration of 0.3 % was expressed as percentage growth of control cultures in the presence of 10% AB serum. The absolute numbers of colonies in the control culture were 17±4 CFU-GM and 20±4 BFU-E (mean ±SE per 10³ cells plated). Protein concentrations were determined by the Coomassie blue binding assay. Molecular mass markers: bovine serum albumin (BSA, Mᵣ 67,000), ovalbumin (OV, Mᵣ 45,000), chymotrypsinogen (CHYM, Mᵣ 25,000) and lysozyme (LYS, Mᵣ 14,300).

### Figure 3

Purification of HPC growth promoting activity by RP-HPLC. In the absence of serum each RP-HPLC fraction (400 µl) was analyzed for the supportive activity for CFU-GM (x-x) and BFU-E (□-□) growth from monocyte- and T lymphocyte-depleted bone marrow MNC in the presence of HGF (n=3). Colony growth supported by each fraction at a final concentration of 0.3% was expressed as percentage growth of control cultures, obtained in the presence of 10% (v/v) AB serum. Absolute numbers of colonies in the control culture were 12±3 CFU-GM and 16±4 BFU-E (mean±SE per 10³ cells plated) ,Fraction f-32 contained the highest level of supportive activity for both the myeloid and erythroid colony growth, 75±12% and 121±20% (mean percentage growth±SE) respectively.

### Figure 4

SDS/PAGE of RP-HPLC fractions containing biologically active (figure 3) 15 kD protein from 5637 cell conditioned medium.

Of each fraction 20 µl was loaded on a polyacrylamide gel under reducing conditions and proteins were silver stained.

Molecular mass markers (Bio-Rad. Richmond, CA) were phosphorylase b (M, 92,500), bovine serum albumin (M, 66,200), ovalbumin (Mᵣ 45,000), carbonic anhydrase (Mᵣ 31,000), soybean trypsin inhibitor (Mᵣ 21,500, lysozyme (Mᵣ 14,400) and the low Mᵣ marker (Pierce Chemical Company, Rockford, IL) aprotinin (Mᵣ 6,500).

### Figure 5

Amino acid sequence analysis of HPC growth supporting fractions (f) purified from 5637 cell conditioned medium.

Amino acid sequences were determined by Edman degradation with an on-line Applied Biosystems 477A/120A sequencer. Since cysteine residues cannot be detected unless modified, their presence was deducted from the absence of any detectable amino acid signal at such position.

### Figure 6

Neutralization of colony growth with anti-huALP antibodies. Before initiation of the colony assay AB serum (AB, 1% v/v), 5637 CM (CM, 10 % v/v), purified natural ALP (f-32, 0.1 % v/v), rALP (10 ng/ml) were incubated at 37^{°}C during 2 h in the presence of control medium or 40 µg of polyclonal IgG anti-huALP antibodies, sufficient to neutralize 400 ng rALP.

Colony growth was expressed as percentage growth of control culture, obtained in the presence of 10% (v/v) AB serum. The number of CFU-GM and BFU-E in the control culture was respectively 27 and 28 per 10³ cells plated respectively. In the presence of anti-huALP antibodies colony growth supported by 5637 CM, fraction f-32 or rALP, but not by 1 % (v/v) AB serum, was completely neutralized.

### Figure 7

Growth support of CFU-GM and BFU-E colonies from monocyte- and T lymphocyte-depleted mononuclear bone marrow cells in the presence of multiple HGF by proteinase inhibitors. In the absence of serum the two separate domains of rALP, the NH₂-terminal domain D-1 and the COOH-terminal domain D-2, were added separately or in combination at molar concentrations equivalent to rALP (100 ng/ml). Colony growth is expressed as percentage of growth supported by rALP (100 ng/ml) (mean±SE, n=3).

### Figure 8

Inhibition of the suppressive effect of CD34 negative accessory cells on HPC growth of purified CD34 positive cells in the absence of serum by proteinase inhibitors. In the presence of HGF the CD34 positive cells were cultured with increasing numbers of CD34 negative cells up to a ratio 1:16 supplemented with IMDM, rALP (100 ng/ml), CM (10 % v/v), AB serum (10% v/v) or α-1 PI (2 µg/ml). The growth of CFU-GM and BFU-E is expressed as percentage growth of control culture, obtained in the presence of 10% (V/V) AB serum in the absence of CD34 negative cells. The results of two experiments (A and B) are shown.

### REFERENCES

1. Sieff, C. A., S.C. Ekern, D.G. Nathan, and J.W. Anderson. 1989. Combinations of recombinant colony-stimulating factors are required for optimal hematopoietic differentiation in serum-deprived culture. *Blood* 73:688-693.
2. Barnes, D., and G. Sato. 1980. Methods for growth of cultured cells in serum-free medium. *Anal*. *Biochem*. 102:255-270.
3. Correa, P.N., and A.A. Axelrad. 1991. Production of erythropoietic bursts by progenitor cells from adult human peripheral blood in an improved serum-free
   medium: Role of insulinlike growthfactor 1.*Blood* 78:2823-2833.
4. Schwartz, G.N., W.R. Hudgins, and J.F. Perdue. 1993. Glycosylated insulin-like growth factor IT promoted expansion of granulocyte-macrophage colony-forming cells in serum-deprived liquid cultures of human peripheral blood cells. *Exp*. *Hematol*. 21:1447-1454.
5. Lansdorp, P.M., and W. Dragowska. 1992. Long-term erythropoiesis from constant numbers of CD34⁺ cells in serum-free cultures initiated with highly purified progenitor cells from human bone marrow. *J*. *Exp*. *Med*. 175:1501-1509.
6. Mayani, H., W. Dragowska, and P.M. lansdorp. 1993. Characterization of functionally distinct subpopulations of CD34⁺ cord blood cells in serum-free long-term cultures supplemented with hematopoietic cytokines. *Blood* 82:2664-2672.
7. Krystal, G., V. Lam, W. Dragowska, C. Takahashi, J. Appel, A. Gontier, A. Jenkins, H. Lam, L. Quon, and P. Lansdorp. 1994. Transforming growth factor β1 is an inducer of erythroid differentiation. *J*. *Exp*. *Med*. 180:851-860.
8. Cohn, E.J. 1947. Separation of blood into fractions of therapeutic value. *Ann*. *Int*. *Med*. 26:341-352.
9. Chen, R.F. 1967. Removal of fatty acids from serum albumin by charcoal treatment.
   *J*. *Biol*. *Chem*. 242:173-181.
10. Goselink, H.M., D.E. Williams, W.E. Fibbe, H.W. Wessels, G.C. Beverstock, R. Willemze, and J.H.F. Falkenburg. 1992. Effect of mast cell growth factor (c-kitligand) on clonogenic leukemic precursor cells. *Blood* 80:750-757.
11. Kramps, J.A., C. Franken, and J.H. Dijkman. 1984. ELISA for quantitative measurement of low-molecular-weight bronchial protease inhibitor in human sputum. *Am*. *Rev*. *Respir*. *Dis*. 129:959-963.
12. Ruck, A., E. Jakobson, S. Björkman, and S. Paulie. 1994. Adaptation of human bladder carcinoma cell lines to serum-free growth. Evidence for autocrine growth stimulation. *Anticancer Res*. 14:55-60.
13. Kaashoek, J.G.J., R. Mout, J.H.F. Falkenburg, R. Willemze, W.E. Fibbe, and J.E. Landegent. 1991. Cytokine production by the bladder carcinoma cell line 5637: Rapid analysis of mRNA expression levels using a cDNA-PCR procedure. *Lymph*. *& Cytok*. *Res*. 10:231-235.
14. Van Damme, J., S. Cayphas, J. Van Snick, R. Conings, W. Put, J.-P. Lenaerts, R.J. Simpson, and A. Billiau. 1987. Purification and characterization of human fibroblast-derived hybridoma growth factor identical to T-ceff-derived B-wu stimulatory factor-2 (interleukin-6). *Eur*. *J*. *Biochem*. *168:543-550*.
15. Van Damme, J., G. Opdenakker, R.J. Simpson, M.R. Rubira, S. Cayphas, A. Vink,
   A. Billiau, and A. Van Snick. 1987. Identification of the human 26-kD protein, interferon β₂ (IFN-B2), as a B cell hybridoma/plasmacytoma growth factor induced by Interleukin-1 and Tumor Necrosis Factor. *J*. *Exp*. *Med*. 165:914-919.
16. Van Damme, J., C. Uyttenhove, F. Houssiau, W. Put, P. Proost, and J. Van Snick. 1992. Human growth factor for murine interleukin (IL)-9 responsive T cell lines: coinduction with IL-6 in fibroblasts and identification as LIF/HILDA. *Eur*. *J*. *Immunol*. 22:2801-2808.
17. Proost, P., A. Wuyts, R. Conings, J.-P. Lenaerts, A. Billiau, G. Opdenakker, and J. Van Damme,. 1993.. Human and bovine granuulocyte chemotactic protein-2: complete amino acid sequence and functional characterization as chemokines. *Biochemistry* 32:10170-10177.
18. Thompson, R.C., and K. Ohlsson. 1986. Isolation, properties, and complete amino acid sequence of human secretory leukocyte protease inhibitor, a potent inhibitor of leukocyte elastase. *Proc*. *Nati*. *Acad*. *Sci*. *USA* 83:6692-6696.
19. Heinzel, R., H. Appelhans, G. Gassen, U. Seemüller, W. Machleidt, H. Fritz, and G. Steffens. 1986. Molecular cloning and expression of CDNA for human antileukoprotease from cervix uterus. *Eur*. *J*. *Biochem*. 160:61-67.
20. Franken, C., C.J.L.M. Meijer, J.H. Dijkman. 1989. Tissue distribution of antileukoprotease and lysozyme in humans. *J*. *Histochem*. *& Cytochem*. 37:493-498.
21. Kramps, J.A., L.N.A. Willems, R. De Water, C. Franken, and J.H. Dijkman. 1991. Bronchial secreting cells and antileukoprotease. In Endothelial and Mucus secreting cells. E. Pozzi, editor, Estratto da, Meetings on pathophysiology of pulmonary cells, Milano, I. 235-245..
22. Björk, P., L. Axelsson, M. Bergenfeldt, and K. Ohlsson. 1988. Influence of plasma protease inhibitors and the secretory leucocyte protease inhibitor on leucocyte elastase induced comsumption of selected plasma proteins *in vitro* in man. *Scan*. *J*. *Clin*. *Lab*. *Invest*. 48:205-211.
23. Laouar, A., J. Wietzerbin, and B. Bauvois. 1993. Divergent regulation of cell surface protease expression in HL-60 cells differentiated into macrophages with granulocyte macrophage colony stimulating factor or neutrophils with retinoic acid. *Int*. *Immunol*. 5:965-973.
24. Deane, D., L. Inglis, and D. Haig. 1995. The 175 antigen expressed on myeloid and erythroid cells during differentiation is associated with serine protease activity. *Blood* 85:1215-1219.
25. Shipp, M.A., and A.T. Look. 1993. Hematopoietic differentiation antigens that are membrane-associated enzymes: cutting is the key! *Blood* 82:1052-1070.
26. Hastka, J. 1988. The importance of granulocyte elastase in haematological diagnosis. *Blut* 57:69-75

**Table 1**

| **Effect of 5637 conditioned medium on colony growth of monocyte- and T lymphocyte-depleted bone marrow mononuclear cells in the absence of serum** | | | |
|---|---|---|---|
| **plated Condition** | | **Colonies per** 103 **cefls** | |
| | | CFU-GM | **BFU-E** |
| Serumfree medium 5637 CM (v/v) | | 4 ± 1 | 0 |
| | 10% | 28 ± 3 | 17 ± 2 |
| | 3% | 18 ± 3 | 13 ± 3 |
| | 1% | 9 ± 2 | 7 ± 2 |
| **AB** serum (v/v) | 10% | 21 ± 2 | 20 ± 2 |

All cultures were performed in the presence of optimal concentrations HGF (GM-CSF 10 ng/ml, G-CSF 10 ng/ml, IL-3 50 ng/ml, SCF 50 ng/ml) and EPO 1 IU/ml).

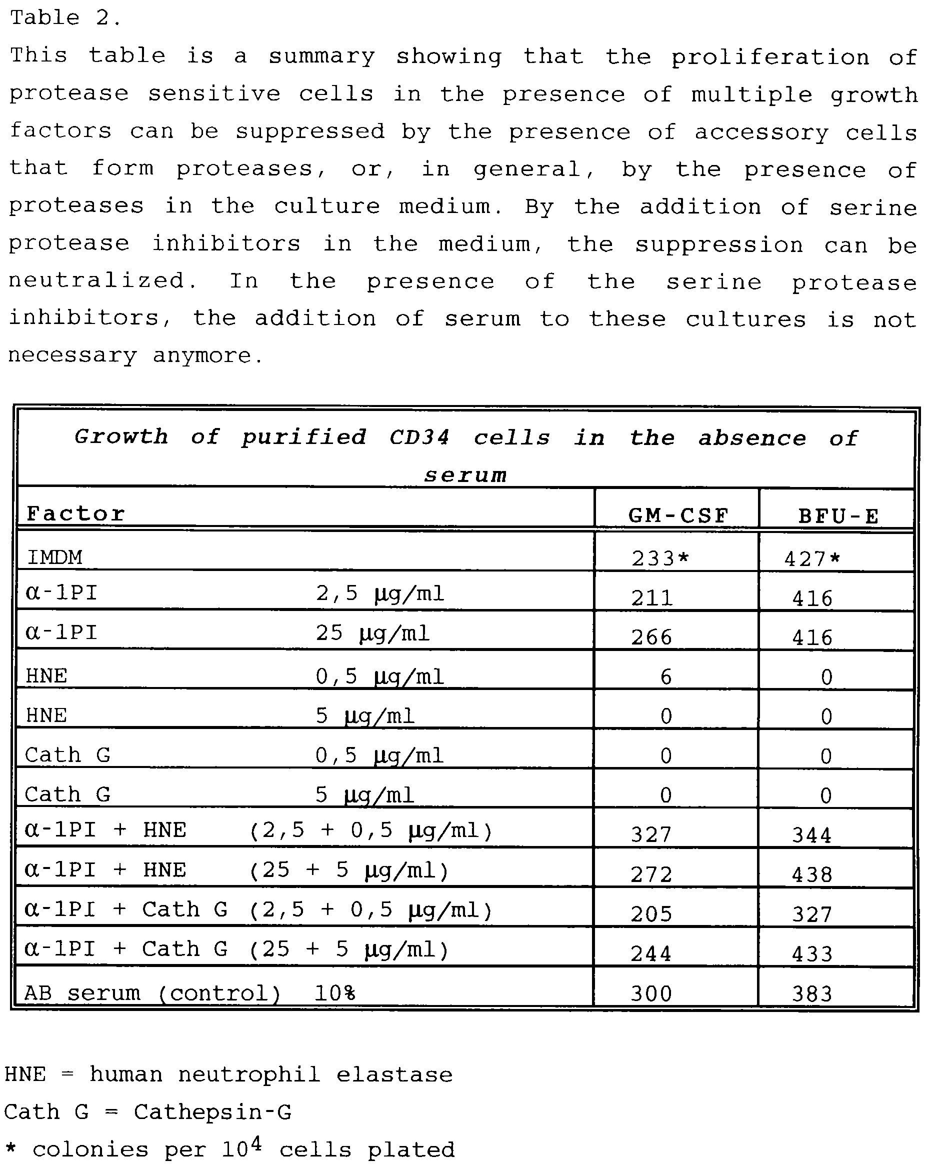

## Claims

1. A method for culturing cells which can be inhibited by the presence of proteases comprising bringing the cells into a container suitable for culturing together with a culture medium and growth factors, whereby a protease inhibitor is added to said culture medium.

2. A method according to claim 1, whereby the growth factors are added to the medium in a purified form.

3. A method according to claim 1 or 2 whereby the protease inhibitor is a seine protease inhibitor.

4. A method according to claim 3 whereby the inhibitor is antileukoproteinase or a functional fragment or derivative thereof.

5. A method according to any one of the aforegoing claims whereby the cells are hematopoietic cells.

6. A method according to claim 5 whereby the cells comprise hematopoietic stem cells and/or hematopoietic progenitor cells.

7. Use of a protease inhibitor in the culturing of cells.

8. Use of a serpin in the culturing of cells.

9. Use of antileukoproteinase in the culturing of cells.

10. Use according to any one of claims 7-9 in the culturing of hematopoietic stem cells and/or progenitor cells.

11. Use according to claim 10 in the production of said cells for reconstitution of a hematopoietic system.

12. Elastase antagonist for use as a pharmaceutical.

13. Elasase antagonist for use in the treatment of myeloid leukemias.

14. Elastase antagonist for use according to claim 12 or 13 whereby the elastase is human neutrophil elastase or a functional equivalent thereof.

15. Elastase antagonist for use according to any one of claims 12-14 which is a serpin or a fragment or derivative thereof.

16. Elastase antagonist for use according to claim 15 which is α1-PI or a functional equivalent, fragment or derivative thereof.

17. Cathepsin G antagonist for use as a pharmaceutical.

18. Cathepsin G antagonist for use in the treatment of myeloid leukemias.

19. Cathepsin G antagonist which is a serpin or a fragment or derivative thereof.
